Europäisches Patentamt

European Patent Office

Office européen des brevets

(19)

(11) Publication number: **0 460 650 A2**

## EUROPEAN PATENT APPLICATION

(12)

(21) Application number: 91109205.4

(22) Date of filing: 05.06.91

(51) Int. Cl.⁵: **G01N 33/76, G01N 33/78,** //G01N33/58,C12Q1/28

(30) Priority: **07.06.90 FI 902856**

(43) Date of publication of application: **11.12.91 Bulletin 91/50**

(84) Designated Contracting States: **AT DE FR GB IT**

(71) Applicant: **LABSYSTEMS OY Pulttitie 8 SF-00881 Helsinki(FI)**

(72) Inventor: **Tuuminen, Tamara Kuparitie 14-16 C SF-00440 Helsinki(FI)** Inventor: **Käpyaho, Kirsti Nallenpolku 2 B 18 SF-02110 Espoo(FI)**

(74) Representative: **Leiser, Gottfried, Dipl.-Ing. et al Patentanwälte Prinz, Leiser, Bunke & Partner Manzingerweg 7 W-8000 München 60(DE)**

(54) **Method for the determination of an analyte.**

(57) The invention relates to a method for the determination of thyrotropin, tyroxin or free thyroxin from dried blood samples by means of fluorometric immunoassays. Horseradish peroxidase is used as the enzyme and 3-p-hydroxyphenylpropionic acid as the substrate. The assay is performed in multicuvette format (e.g. microtitration plates), and is rapid and accurate. It is suitable for high capacity testing with automatic processing and measurements.

EP 0 460 650 A2

TECHNICAL FIELD

The present invention relates to fluorometric determination of an analyte from blood samples dried on a filter paper. The analyte to be determined is thyrotropin, thyroxin, or free thyroxin. The determination is preferably performed in multicuvette format (e.g. microtitration plates and is especially suitable for the screening of congenital hypothyreosis from neonatal blood samples impregnated on filter paper.

BACKGROUND

A variety of methods for the determination of human thyrotropin (hTSH) from dried blood samples are known (e.g. Irie, H., Enomoto, K. and Naruse H., Measurement of thyroid stimulating hormone in dried blood spot, Lancet 11, 1975, 1233; Torresani, T., Qui-Qing, Illig, R., Thyrotropin enzyme-immunoassay in dried blood spots: a spectrophotometric method for neonatal thyroid screening, J. Clin. Chem. Clin. Biochem 1986, 24, 199-203; Torresani, T. and Scherz, R., Thyroid screening of neonates without use of radioactivity: evaluation of time-resolved fluoroimmunoassay of thyrotropin, Clin. Chem. 1986, 32, 1013-16; Woodhead, J. S., Siddle K., Weeks J., Immunochemiluminometric assay of thyrotropin in filter paper blood spots, Adv. Neonatal Screening 1987, 149-152; Ishikawa, E., Hashida, S., Umehashi H., Hayashida, T., Mori T., Imura, H., Ogawa H., Highly sensitive sandwich enzyme immunoassay of human thyroid stimulating hormone (hTSH) in dried blood on filter paper discs for mass screening of neonatal hypothyroidism, Adv. Neonatal Screening 1987, 121-122; Volbracht, L., Beyer, P., Weber, B., Helge, H., TSH measurements from blood spots on filter paper by enzyme immunoassay (EIA) alternative to RIA as screening procedure for neonatal hypothyroidism, Adv. Neonatal Screening 1987, 153-155). Such methods are used for the mass screening of neonatal hypothyroidism.

Most of the commercial methods available are radio-immunoassays. They have the drawback that radioactive materials have to be handled.

There are also non-isotopic commercial assays, but many of them are not feasible for mass screenings in big laboratories with a throughput of 1000-1500 samples daily.

There are also non-isotopic commercial assays on microplates meant to be used for mass screenings (e.g. those manufactured by Wallac, Turku, Finland, and by Fujirebo, Tokyo, Japan). These tests demand, however, rather long incubation times (4 hours or overnight), and are therefore less suitable for rapid automated screenings.

Fluorometric enzyme immuno assays (FEIA) are also known for the detection of some substances. The sensitivity and measuring range of such methods are generally significantly better than those of absorbtiometric methods. Sensitive fluorogenic substrates exist for all enzymes commonly used in immuno assays (see e.g. Ishikawa, E., Imagawa, M., Hashida, S., Yoshitake, S., Hamaguchi, Y. and Ueno, T., Enzyme-labeling of antibodies and their fragments for enzyme immunoassays and immunohistochemical staining, J. Immunoassay 1983, 4: 209-327).

Horseradish peroxidase is one common enzyme used in immunoassays. The first comprehensive publication concerning fluorogenic substrates for horseradish peroxidase was published already in 1980 (Zaitsu, K. and Ohkura, Y., New fluorogenic substrates for horseradish peroxidase: rapid and sensitive assay for hydrogen peroxide and the peroxidase, Anal. Biochem. 1980, 109: 109-13)/ 3-p-Hydroxyphenylpropionic acid (HPPA) proved to be the most sensitive of the hydrogen donating substances tested.

Later HPPA was succesfully used in sandwich FEIAs for the detection of different substances such as growth hormone, insulin, alpha-fetoprotein and chronionic gonadotropin (e.g. Hashida, S., Nakagawa, K., Yoshitake, S., Imagawa, M., Ishikawa, E., Endo, Y, Ohtaki, S., Ichioka Yu., Nakajima K., A highly sensitive sandwich enzyme immunoassay of human growth hormone in serum using affinity purified anti-human growth hormone, Fab'-horseradish peroxidase conjugate, Anal. Lett. 1983, 16: 31-44; Zaitsu, K., Nakashima, K., Akiyama, S. and Ohkura, Y., Sensitive fluorogenic substrate for peroxidase and its application to enzyme-immunoassays, J. Pharm. Dyn. 1982, 5: 20; Imagawa, M., Hashida, S., Ishikawa, E., Niitsu, Y., Urushizaki, I., Kanazawa, R., Tashibana, S., Nakazawa, N. and Ogawa H., Comparison of beta-D-galactosidase from Escherichia coli and horseradish peroxidase as labels of anti-human ferritin Fab' by sandwich enzyme immunoassay technique, J. Biochem. 1984, 96: 659-64; Imagawa, M., Hashida, S., Ohta Y. and Ishikawa, E., Evaluation of beta-D-galactosidase from Escherichia coli and horseradish peroxidase as labels by sandwich enzyme immunoassay technique, Ann. Clin. Biochem. 1984, 21: 310-17).

The known FEIAs were performed in test tubes and using separate pearls as the solid phase. The procedures used are not suitable for automatic assays.

GENERAL DESCRIPTION OF THE INVENTION

The present invention comprises an immunochemical method for the measurement of thyrotropin, thyroxin or free thyroxin from dried blood samples by means of fluorometric enzyme immunoassay using horseradish peroxidase as the enzyme and 3-p-hydroxyphenylpropionic acid as the fluorogenic substrate. The determination is suitable to be performed in multicuvette format (e.g. microtitration plates). The invention and some of its preferable embodiments are defined in detail in the enclosed claims.

The method is reliable, accurate and rapid, and it can be easily automated. It is also possible to perform the quantitative determination of TSH over a very wide dynamic range.

An additional advantage of the method is that it can be run together with a fluorometric phenylketonuria test, e.g. such as described in Gerasimova, N., Stelkova, I., Tuuminen, T., Fluorometric method for phenylalanine microplate assay adapted for phenylketonuria screening, Clin. Chem. 1989, 35, 2112-15.

## DETAILED DESCRIPTION OF THE INVENTION

According to the invention, the determination is carried out in a well (e.g. microtitration plate) precoated with an antibody to the analyte, or with the analyte itself, or with their derivative.

The analyte is located in a blood spot dried on a filter paper. A disk containing the analyte is either pre-eluted in a buffer which is then used as the sample, or, preferably, the disk is applied directly into the coated well, and the elution is performed simultaneously with the immunochemical reaction. The reaction buffer is e.g. a phosphate buffer or a 2-[4-(2-hydroxyethyl)-1-piperazinyl]-ethanosulfonic acid (HEPES) buffer or a tris (hydroxymethyl) aminomethane (Tris) buffer containing salt and a protein (such as albumin or gelatin or casoin) and/or serum additive, and (in the case of one-step immunochemical reaction) HRP conjugated with an antibody for the analyte, or with the analyte itself, or with their derivative. The immunochemical reaction may also be performed in two steps, in which case the first incubation is performed in the absence of HRP conjugate, the well is washed, and the second incubation is performed with HRP conjugate present.

After incubation the well is washed. 3-p-Hydroxyphenylpropionic acid (HPPA) in buffer with pH preferably about 7.5 -8.5, and hydrogen peroxide are then added into the well, the mole ratio HPPA/$H_2O_2$ being preferably about 12.5 -13.2, The buffer is e.g. tris(hydroxymethyl) aminomethane (Tris).

The well is then incubated for carrying out the substrate reaction. The reaction is stopped with a buffer with pH about 10.0 - 11.0, preferably about 10.3. The stopping buffer is preferably a glycine buffer.

The fluorescence is measured at 405 nm (excitation at 320 nm).

Following examples further illustrate the invention:

### Example 1. Determination of TSH from neonatal blood samples

A single 5-mm disk is punched from filter paper containing the blood sample and put into a microtitration plate well precoated with anti-TSH antibody directed against intact TSH molecule. 200 microl of a second antibody (directed against TSH $\beta$-subunit conjugated with HRP in 0.01 M phosphate buffer containing 0.15 M sodium chloride, 0.1 % Tween 20 and 2.5 % bovine serum is added to the well and incubated for two hours on a shaker at a room temperature. (Alternatively, the conjugate is dilusted in 20 mM HEPES buffer, pH 7.0, containing 1 g/l BSA, 60 ml/l bovine serum, 150 mM sodium chloride and 0.1 % Tween 20). The well is washed four times with phosphate buffered saline containing 0.1 % Tween 20. The substrate reaction is performed by adding 200 $\mu$l of substrate mixture containing 5 aliquots of 7.5 g/l HPPA in 0.1 M TRIS buffer, pH 7.8, and one aliquot of freshly prepared 0.06 % hydrogen peroxide. Substrate mixture is incubated in a shaker for one hour at a room temperature and the reaction is stopped with 100 $\mu$l of 1.5 M glycine buffer, pH 10.3. The fluorescence is measured in a microplate fluorometer at 405 nm (excitation at 320 nm).

Correlation of the assay with two commercial kits was performed by determining TSH from 47 cord blood samples spotted on filter paper. The correlation with cord serum values was calculated using the respective haematocrite values.

Performance of the assay:

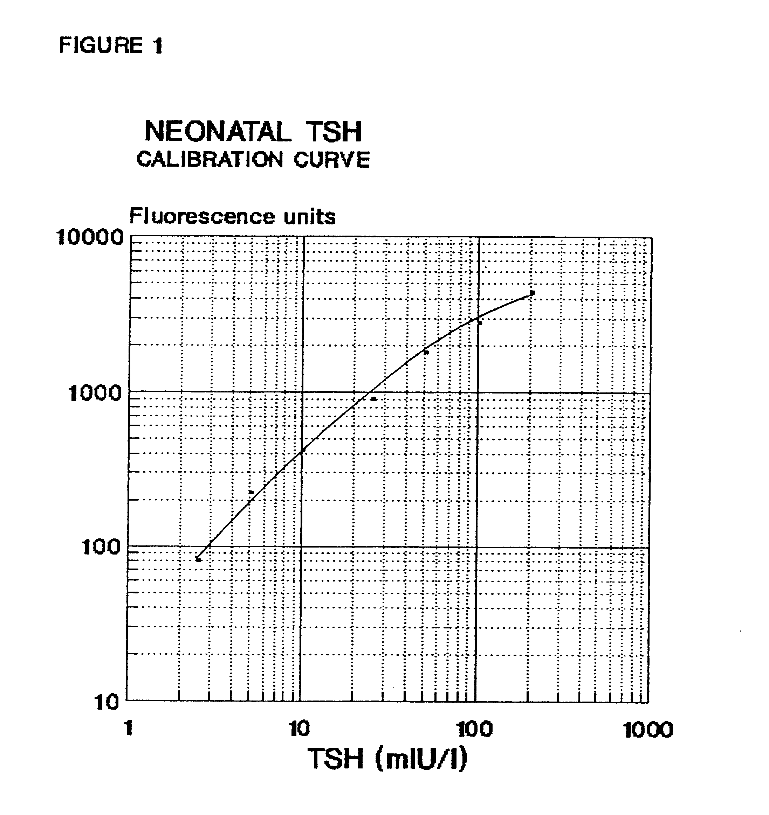

1. The standard curve of the assay is shown in Figure 1.
2. Intra-assay (n = 8) and interassay (n = 13) imprecision:

| TSH mIU/l | Fluorescence (units) | CV % |
|---|---|---|
| Intra-assay: | | |
| 2.5 | 96 + 9 | 9.4 |
| 25 | 1027 + 74 | 7.2 |
| 100 | 3066 + 78 | 2.5 |
| Interassay: | | |
| 2.5 | 92 + 8 | 9.1 |
| 25 | 1047 + 94 | 9.0 |
| 100 | 3121 + 156 | 5.0 |

The dynamic range of the assay was 2.5 to 400 mIU/l. The detection limit was 1.25 mIU/l (the mean fluorescence of the zero standard plus 3 SD).

3. Correlation with commercial kits:

| Methods | Regression equation | Correlation coefficient |
|---|---|---|
| x = EIA Fujirebio | $y = 0.91 \, x + 0.35$ | $r = 0.93$ |
| y = FEIA Labsystems | | |
| x = TR-FIA Wallac | $y = 1.16 \, x - 0.45$ | $r = 0.90$ |
| y = FEIA Labsystems | | |
| x* = IRMA Behring | $y = 1.11 \, x - 0.77$ | $r = 0.95$ |
| y* = FEIA Labsystems | | |

* comparison of actual serum values with calculated ones using haematocrite

4. Interferences

There was no interference by bilirubin at concentrations of 100 $\mu$mol/L and 200 $\mu$mol/L.

Example 2. Determination of T4 from neonatal blood samples

A single 5-mm disk is punched from filter paper containing the blood sample and put into a microplate well precoated with a monoclonal anti-T4 antibody. 200 μl of HRP-T4 conjugate in 0.1 M TRIS buffer, pH 8.6, containing 3 g/l gelatin and 0.4 g/l anilinonaphthalene sulphonic acid (ANS) is added to the well and incubated for two hours on a shaker at room temperature. The well is washed four times with phosphate buffered saline containing 0.1 % Tween 20. The substrate reaction is performed by adding 200 microl of substrate mixture containing 5 aliquots of 7.5 g/l HPPA in 0.1 M Tris buffer, pH 7.8, and one aliquot of fresly prepared 0.06 % hydrogen peroxide. Substrate mixture is incubated in a shaker for one hour at room temperature, and the reaction is stopped with 100 μl of 1.5 M glycine buffer, pH 10.3. The fluorescence is measured in a microplate fluorometer at 405 nm (excitation at 320 nm).

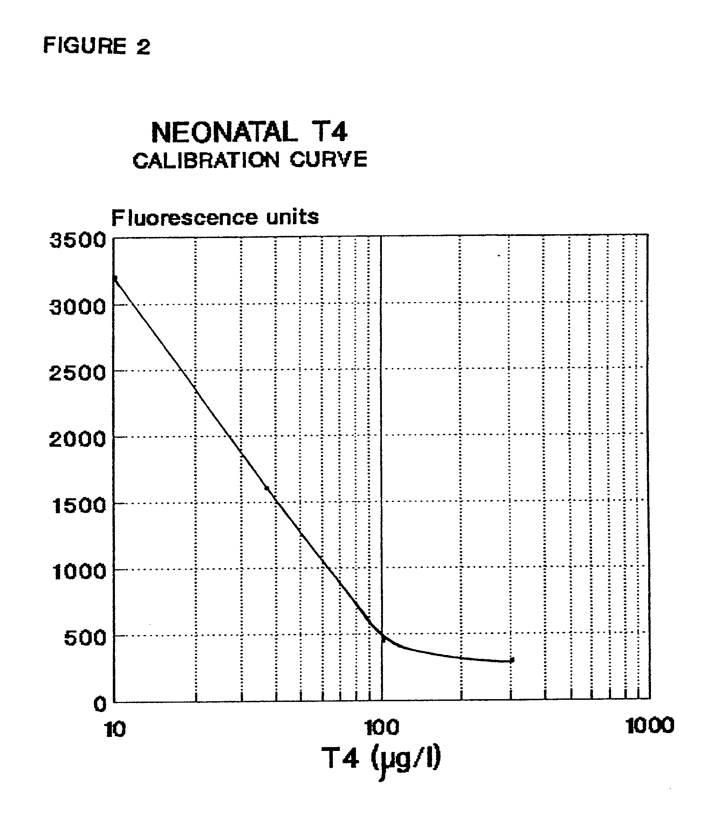

The standard curve of the assay is shown in Figure 2.

**Claims**

1. A Method for the determination of an analyte from a whole blood sample dried on a filter paper, the analyte being selected from the group comprising thyrotropin, thyroxin and free thyroxin, wherein
   - the analyte is eluted into a buffer solution
   - the buffer solution is incubated in a well coated either with an antibody to the analyte, or with the analyte itself or its analogue or their derivative
   - the solution is incubated with horseradish peroxidase conjugated with another antibody to the analyte, or if the well has been coated with an antibody, optionally with horseradish peroxidase conjugated with the analyte itself or its analogue or their derivative, the well is emptied and washed with buffer-detergent solution,
   - 3-p-hydroxyphenylpropionic acid in a buffer with pH preferably about 7.5 - 8.5, most preferably about 7.8, and hydrogen peroxide are added into the well, the mole ratio of 3-p-hydroxyphenylpropionic acid to hydrogen peroxide being preferably about 12 - 15, most preferably about 12.8,
   - the well is incubated for carrying out the substrate reaction,
   - the substrate reaction is stopped with buffer, whose pH is preferably about 10 - 11, most preferably about 10.3, and
   - the fluorescence of the solution is measured.

2. A method according to claim 1, wherein the elution and the first incubation are carried out simultaneously.

3. A method according to claim 1 or 2, wherein the first incubation and the second incubation are carried out simultaneously.

4. A method according to any of claims 1-3, for the determination of thyrotropin, wherein the elution and/or the incubation buffer has the pH preferably about 6.5 - 7.8, and contains a protein, such as albumin, and/or serum additive.

5. A method according to any of claims 1-3 for the dedetermination of thyroxin or free thyroxin, wherein the incubation buffer has the pH preferably about 7.5 - 9.0, and optionally contains a protein, such as gelatin or casein, additive.

## FIGURE 1

NEONATAL TSH
CALIBRATION CURVE

## FIGURE 2

**NEONATAL T4**
CALIBRATION CURVE